# EUROPEAN PATENT APPLICATION

(11) **EP 3 632 329 A1**
(43) Date of publication of application: **08.04.2020**
(21) Application number: 18198803.1
(22) Date of filing: 05.10.2018
(51) Int. Cl.: A61B 8/00

(54) **EXTENSION CABLE FOR AN ULTRASOUND SYSTEM**

(71) Applicant: Koninklijke Philips N.V., 5656 AG Eindhoven (NL)
(72) Inventor: CLAESSENS, Eric Franciscus Johannes, 5656 AE Eindhoven (NL)
(74) Representative: de Haan, Poul Erik

(57) **Abstract**

Extension cable (EXC1, EXC2) for use in an ultrasound system. The extension cable includes a first electrical connector (FEC) for connecting to a corresponding ultrasound transducer connector (UTC), a second electrical connector (SEC) for connecting to a corresponding console connector (CC), an electrical circuit (ECCT), and an electrical cable (ECAB). The electrical circuit (ECCT) provides a multiple-state output (MSOP), the multiple states being voltage levels (V₁, V₂, V₃) corresponding to each of i) the electrical circuit (ECCT) not being electrically connected to the console connector (CC), ii) the electrical circuit (ECCT) being electrically connected to the console connector (CC) and the ultrasound transducer connector (UTC) not being electrically connected to the first electrical connector (FEC), and iii) the electrical circuit (ECCT) being electrically connected to the console connector (CC) and the ultrasound transducer connector (UTC) being electrically connected to the first electrical connector (FEC).

## Description

### FIELD OF THE INVENTION

The invention relates to an extension cable. The extension cable finds particular application in an ultrasound system. In one application the ultrasound system is an ultrasound-based position tracking system and the extension cable is used to connect an ultrasound transducer to the ultrasound-based position tracking system.

### BACKGROUND OF THE INVENTION

In the medical field, ultrasound transducers are increasingly used to gain more information about, or to treat, a patient's anatomy. In this regard, medical devices may be equipped with an ultrasound transducer for use in sensing and actuation applications such as tracking, imaging, and treatment.

In one exemplary application described in more detail in document "A Non-disruptive Technology for Robust 3D Tool Tracking for Ultrasound-Guided Interventions" by Jay Mung, Francois Vignon, and Ameet Jain, in MICCAI 2011, Part I, LNCS 6891, pp. 153-160, 2011, A. Martel, and T. Peters (Eds.), an ultrasound detector is attached to a medical needle and used to track the needle position respective the ultrasound field of a beamforming ultrasound imaging probe based on the timing of ultrasound signals detected by the detector.

Conventionally such ultrasound transducers may be provided with a connector that connects to a corresponding connector mounted on a console. The ultrasound signals may subsequently be processed in the console. However, in such applications there is often a need to provide an extended electrical path between an ultrasound transducer and the console. Extension cables are in general known for this purpose.

### SUMMARY OF THE INVENTION

The present invention seeks to provide an improved extension cable for use in an ultrasound system. The extension cable may be used to electrically connect an ultrasound transducer to a console.

Thereto, an extension cable for use in an ultrasound system is provided. The extension cable includes a first electrical connector for connecting to a corresponding ultrasound transducer connector, a second electrical connector for connecting to a corresponding console connector, an electrical circuit and an electrical cable. The electrical circuit is disposed between the first electrical connector and the electrical cable. The electrical cable is disposed between the electrical circuit and the second electrical connector for extending an electrical connection between the ultrasound transducer connector and the console connector. The electrical circuit is configured to provide a multiple-state output, the multiple states being voltage levels corresponding to each of i) the electrical circuit not being electrically connected to the console connector via the second electrical connector, ii) the electrical circuit being electrically connected to the console connector via the second electrical connector and the ultrasound transducer connector not being electrically connected to the first electrical connector, and iii) the electrical circuit being electrically connected to the console connector via the second electrical connector and the ultrasound transducer connector being electrically connected to the first electrical connector.

The extension cable thus has the ability to determine a connection status of its first and/ or second electrical connectors with their corresponding ultrasound transducer and console connectors. When a conventional extension cable is used to extend a desired path between such a console and ultrasound transducer a user relies upon observing a causality between inputs to the ultrasound transducer and a response observed via the console, to determine that the path has been extended correctly and the system is operating correctly. For example, if the ultrasound transducer is a detector and the console indicates detected ultrasound signal strength, the user relies upon seeing an indication of an expected signal strength on the console in response to an ultrasound test signal. However, such connectors have multiple failure modes including the failure of one or more of its terminals to contact a corresponding terminal in the counterpart connector. The source of such failures can be tricky to identify, because the user may have to initially check that each connector is properly connected, and subsequently verify that the ultrasound test signal was actually sent. Other connector failure modes can result in partial operation of the system and thus may go unnoticed. One example of this is high contact resistance at one or more of the connector terminals. This may result in a diminished yet otherwise apparently normal detected ultrasound signal. Checking the nature of these connections can be time-consuming for the user. Under such circumstances it may be advantageous to have an extension cable that determines the present connection status of the extension cable's connectors with their corresponding ultrasound transducer and console connectors.

According to one aspect the electrical circuit further includes a memory configured to store a count value indicative of a) a number of times that a corresponding ultrasound transducer connector has been connected to the first electrical connector based a number of times the voltage level of the multiple state output transitions to the voltage level corresponding to iii) the electrical circuit being electrically connected to the console connector via the second electrical connector and the ultrasound transducer connector being electrically connected to the first electrical connector, and/ or b) a number of times that a corresponding console connector has been connected to the second electrical connector based on a number of times the voltage level of the multiple state output transitions to the voltage level corresponding to ii) the electrical circuit being electrically connected to the console connector via the second electrical connector and the ultrasound transducer connector not being electrically connected to the first electrical connector.

In this aspect the memory stores number of connection cycles between one or both of the extension cable's connectors and their corresponding ultrasound transducer and console connectors. Advantageously the count of each of these connection cycles may be used to indicate the need to replace the extension cable and thereby reduce the chance of its failure.

In accordance with another aspect a system and an arrangement that include the extension cable are provided.

Further aspects are described with reference to the appended claims. Further advantages from the described invention will also be apparent to the skilled person.

### BRIEF DESCRIPTION OF THE FIGURES

Fig. 1 illustrates an extension cable that includes first electrical connector FEC, second electrical connector SEC, electrical circuit ECCT and electrical cable ECAB.
Fig. 2 illustrates an extension cable that includes first electrical connector FEC, second electrical connector SEC, electrical circuit ECCT, electrical cable ECAB and two measurement terminals TM₁, TM₂.
Fig. 3 illustrates various exemplary multiple-state output MSOP voltage levels V₁, V₂, V₃.
Fig. 4 illustrates an exemplary electrical circuit ECCT that includes an active electronic component TR₁.
Fig. 5 illustrates an exemplary electrical circuit ECCT that includes only passive electrical components.
Fig. 6 illustrates a system SYS that includes extension cable EXC2, console CON and console connector CC.

### DETAILED DESCRIPTION OF THE INVENTION

In order to illustrate the principles of the present invention an extension cable is described with particular reference to an exemplary ultrasound-based position tracking system in which the extension cable connects an ultrasound transducer to a console by means of the extension cable's first and second electrical connectors, together with a corresponding ultrasound transducer connector and a corresponding console connector. It is however to be appreciated that the extension cable finds application in the ultrasound field in general, particularly the medical ultrasound field. Use of the extension cable in ultrasound sensing and actuation application areas such as position tracking, imaging, and treatment is thus also contemplated. Moreover, whilst in some exemplary application areas, reference is made to an ultrasound transducer in the specific form of an ultrasound detector, the term ultrasound transducer should be interpreted more broadly as an ultrasound detector, or an ultrasound emitter, or a device that is capable of both detecting and emitting ultrasound signals, or indeed a device that comprises both an ultrasound emitter and an ultrasound detector.

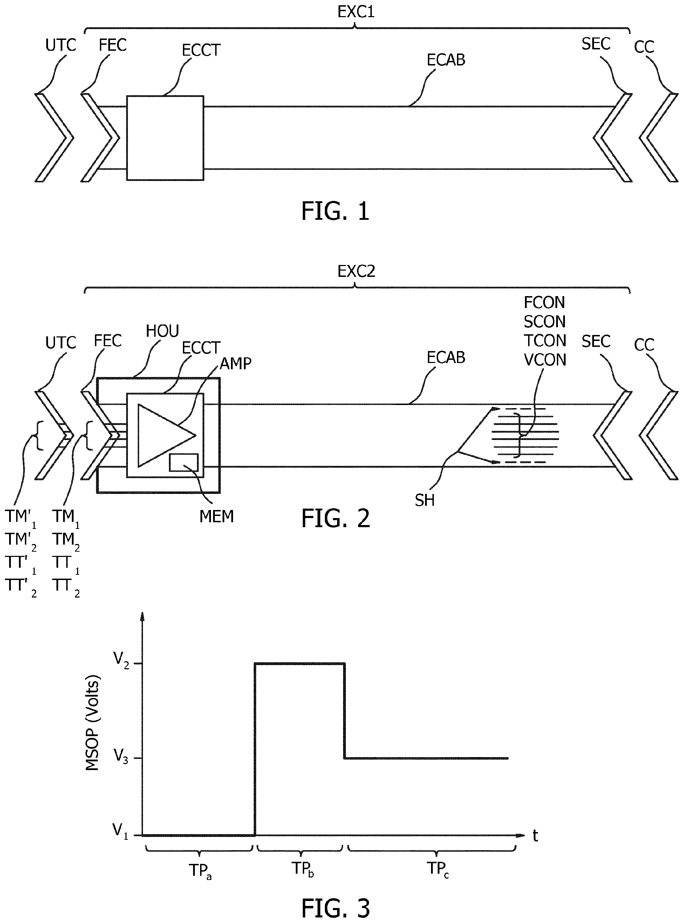

Fig. 1 illustrates an extension cable that includes first electrical connector FEC, second electrical connector SEC, electrical circuit ECCT and electrical cable ECAB. Extension cable EXC1 includes first electrical connector FEC that is suitable FEC for connecting to corresponding ultrasound transducer connector UTC. Second electrical connector SEC is suitable for connecting to a corresponding console connector CC. Extension cable EXC1 is suitable for use in an ultrasound system in that its electrical connectors FEC, SEC are suitable for carrying electrical signals having frequencies that are typically used in ultrasound applications, i.e. up to a frequency of approximately 1 MHz in some instances, or up to approximately 2 - 10 MHz frequencies in other instances. Extension cable EXC1 also includes electrical circuit ECCT and electrical cable ECAB. Electrical cable ECAB may include electrical conductors, not shown in Fig. 1, for communicating ultrasound signals between electrical connectors FEC and SEC. Electrical circuit ECCT is disposed between the first electrical connector FEC and the electrical cable ECAB, and electrical cable ECAB is disposed between electrical circuit ECCT and second electrical connector SEC in order to extend an electrical connection between ultrasound transducer connector UTC and console connector CC. Moreover, electrical circuit ECCT is configured to provide a multiple-state output MSOP, the multiple states being voltage levels V₁, V₂, V₃ corresponding to each of three connection states. The first connection state is that i) the electrical circuit ECCT is not electrically connected to console connector CC via second electrical connector SEC. The second connection state is that ii) electrical circuit ECCT is electrically connected to console connector CC via second electrical connector SEC and ultrasound transducer connector UTC is not electrically connected to first electrical connector FEC. The third connection state is that iii) electrical circuit ECCT is electrically connected to console connector CC via second electrical connector SEC and ultrasound transducer connector UTC is electrically connected to first electrical connector FEC.

Various types of connector are suitable for use as first electrical connector FEC and second electrical connector SEC. Connectors types such as USB, IEEE-488 GPIB, D-type, RJ-type, DB-type, coaxial and 8P8C are some non-limiting examples that are contemplated. The male or female counterpart is thus suitable for use as corresponding ultrasound transducer connector UTC and corresponding console connector CC.

Various configurations of electrical circuit ECCT are contemplated for use in Fig. 1 to provide multiple-state output MSOP. Electrical circuits that include one or more active electronic component are contemplated. Electrical circuits that include only passive electrical components such as resistors, capacitors or diodes, are also contemplated.

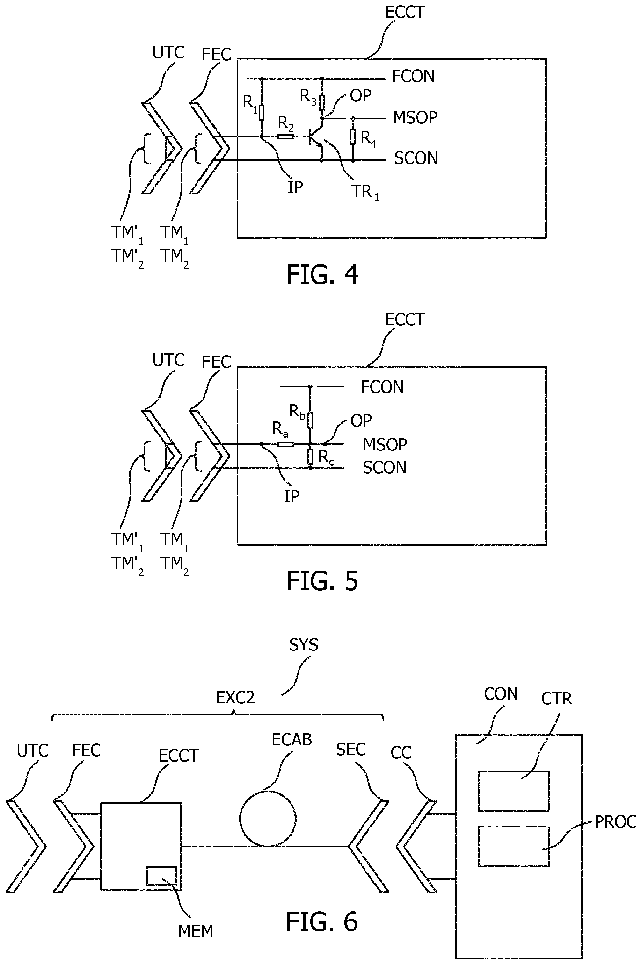

Fig. 4 illustrates an exemplary electrical circuit ECCT that includes an active electronic component TR₁. Active electronic component TR₁ is a transistor in this exemplary configuration and is configured to operate as an electrical switch. When connected, first electrical conductor FCON and second electrical conductor SCON that form part of electrical cable ECAB provide a potential difference from the console connector CC to electrical circuit ECCT by means of corresponding terminals in console connector CC and second electrical connector SEC. For the purposes of explanation only, reference is made to an exemplary potential difference of 5V and the emitter of transistor TR₁ may be considered to be connected to 0V reference. Electrical circuit ECCT provides multiple-state output MSOP voltage levels V₁, V₂, V₃ at electrical output OP. With additional reference to Fig. 3, which illustrates various exemplary multiple-state output MSOP voltage levels V₁, V₂, V₃, the exemplary electrical circuit ECCT of Fig. 1 operates in the following manner. In a first connection state; i.e. i) the electrical circuit ECCT is not electrically connected to console connector CC via second electrical connector SEC, a voltage level V₁ is provided at output OP. Voltage level V₁ may for example be 0V as illustrated in Fig. 3 during time period TPₐ due to the absence of any power to electrical circuit ECCT. Thus, the voltage level V₁ corresponding to the first connection state, i), is provided if the first electrical conductor (FCON) and the second electrical conductor (SCON) is not electrically connected to its corresponding terminal in the console connector (CC). With reference to Fig. 4, electrical circuit ECCT may subsequently be electrically connected to console connector CC via second electrical connector SEC. When connected, first electrical conductor FCON and second electrical conductor SCON that form part of electrical cable ECAB are configured to provide the exemplary 5V potential difference from the console connector CC to the electrical circuit ECCT by means of corresponding terminals in console connector CC and second electrical connector SEC. In the second connection state, i.e. ii) electrical circuit ECCT is electrically connected to console connector CC via second electrical connector SEC and ultrasound transducer connector UTC is not electrically connected to first electrical connector FEC, a voltage level V₂ is provided at output OP. When electrical circuit ECCT is electrically connected to console connector CC via second electrical connector SEC, resistors R₁ and R₂ pull-up the base of transistor TR₁ to the exemplary 5V potential of first electrical conductor FCON, which switches transistor TR₁ on. Output OP is set to a voltage level V₂, which is determined in part by the relative values of resistors R₃ and R₄ in resistor divider R₃ - R₄ and in part by the current flowing from collector to emitter of TR₁ in its on state. In this second connection state, corresponding voltage V₂ may for example be set based on these factors to approximately 2.5V. With reference to Fig. 3, voltage level V₂ may be provided during time period TP_{b}. In the third connection state, i.e. iii) electrical circuit ECCT is electrically connected to console connector CC via second electrical connector SEC and ultrasound transducer connector UTC is electrically connected to first electrical connector FEC, voltage level V₃ is provided at output OP. With reference to Fig. 4, first electrical connector FEC may include two or more measurement terminals TM₁, TM₂ for connecting to corresponding terminals TM'₁, TM'₂ in ultrasound transducer connector UTC. Measurement terminals TM₁ is connected to electrical circuit ECCT input IP. Corresponding terminals TM'₁, TM'₂ in ultrasound transducer connector UTC are electrically connected together. Thus when ultrasound transducer connector UTC is electrically connected to first electrical connector FEC, measurement terminals TM₁, TM₂ are electrically connected together, causing input IP and thus the base of transistor TR₁ to be connected to 0V. Transistor TR₁ thus switches off, causing the potential of output OP to be determined by the relative values of resistors R₃ and R₄. In this configuration, input IP is therefore configured to detect whether the ultrasound transducer connector UTC is electrically connected to the first electrical connector FEC. In this third connection state, corresponding voltage V₃ may for example be approximately 1V, as illustrated in Fig. 3 during time period TP_{c}. Voltage level V₃ corresponding to the third connection state, is therefore generated based on a value of an impedance measured between the two measurement terminals TM₁, TM₂.

Clearly the absolute values of voltage levels V₁, V₂, V₃ may be adjusted by changing the potential difference applied to the circuit, or by changing the resistor values, or the on-state resistance of transistor TR₁. Moreover, a pullup resistor in console connector CC may be connected between an input that connects to electrical circuit ECCT output OP and the exemplary 5V potential of first electrical conductor FCON to further adjust the absolute values of voltage levels V₂ and V₃ by virtue of its effect as a resistive divider. Moreover, whilst in the example circuit of Fig. 4 there is no local power source permanently attached to electrical circuit ECCT on extension cable ECAB, which reduces the technical complexity of the extension cable, in alternative configurations it is contemplated to use such a local power source. The voltage provided by such a local power source to electrical circuit ECCT may clearly also determine the absolute values of voltage levels V₁, V₂, V₃. Moreover, it is to be appreciated that the example electrical circuit ECCT of Fig. 4 is only exemplary, and alternative circuits with alternative, or additional active components such as FET transistors, a microprocessor and so forth may also be used.

In an alternative implementation to that of Fig. 4, Fig. 5 illustrates an exemplary electrical circuit ECCT that includes only passive electrical components. The electrical circuit ECCT of Fig. 5 may be used in place of ECCT in Fig. 1. With reference to Fig. 5, passive components in the form of resistors Rₐ, R_{b}, R_{c}, are connected as resistive dividers in order to provide the exemplary aforementioned voltage levels V₁, V₂ and V₃. In a first connection state; i.e. i) the electrical circuit ECCT is not electrically connected to console connector CC via second electrical connector SEC, a voltage level V₁ is provided at output OP. Voltage level V₁ may for example be 0V as illustrated in Fig. 3 during time period TPₐ due to the absence of any power to electrical circuit ECCT. Thus the voltage level V₁ corresponding to the first connection state, i), is provided if the first electrical conductor FCON and the second electrical conductor SCON is not electrically connected to its corresponding terminal in the console connector CC. In a second connection state, i.e. ii) electrical circuit ECCT is electrically connected to console connector CC via second electrical connector SEC and ultrasound transducer connector UTC is not electrically connected to first electrical connector FEC, a voltage level V₂ is provided at output OP. Voltage level V₂ may for example be approximately 2.5V as illustrated in Fig. 3 during time period TP_{b}. With reference to Fig. 5, in this state, voltage V₂ at output OP is determined by the resistive divider comprising R_{b} and R_{c}. In a third connection state, i.e. iii) electrical circuit ECCT is electrically connected to console connector CC via second electrical connector SEC and ultrasound transducer connector UTC is electrically connected to first electrical connector FEC, a voltage level V₃ is provided at output OP. Voltage level V₃ may for example be approximately 1V as illustrated in Fig. 3 during time period TP_{c}. With reference to Fig. 5, first electrical connector FEC may include two or more measurement terminals TM₁, TM₂ for connecting to corresponding terminals TM'₁, TM'₂ in ultrasound transducer connector UTC. Corresponding terminals TM'₁, TM'₂ in ultrasound transducer connector UTC are electrically connected together. Thus when ultrasound transducer connector UTC is electrically connected to first electrical connector FEC, input IP is connected to SCON by virtue of measurement terminals TM₁, TM₂ being electrically connected together. This causes the voltage of output OP to be determined by the parallel combination of Rₐ and R_{c}, and the resistive divider this forms with R_{b}. Thus, the voltage level V₃ corresponding to the third connection state, is in electrical circuit ECCT in Fig. 5 provided based on a value of an impedance measured between the two measurement terminals TM₁, TM₂. Clearly the absolute values of voltage levels V₁, V₂, V₃ in the implementation of Fig. 5 may be adjusted as mentioned above in relation to Fig. 4, i.e. by changing the potential difference applied to the circuit, or the resistor values Moreover, a pullup resistor in console connector CC may be connected between an input that connects to electrical circuit ECCT output OP and the exemplary 5V potential of first electrical conductor FCON to further adjust the absolute values of voltage levels V₂ and V₃ by virtue of its effect as a resistive divider. Moreover, it is to be appreciated that the example electrical circuit ECCT of Fig. 5 is only exemplary, and alternative circuits with alternative, or additional passive components such as capacitors, diodes and so forth may also be used.

Fig. 2 illustrates an extension cable EXC2 that includes first electrical connector FEC, second electrical connector SEC, electrical circuit ECCT, electrical cable ECAB and two measurement terminals TM₁, TM₂. Measurement terminals TM₁, TM₂ may, as described above, be used to connect to corresponding terminals TM'₁, TM'₂ in ultrasound transducer connector UTC. Moreover, as described above, electrical circuit ECCT in Fig. 2 provides voltage level V₃ corresponding to iii) electrical circuit ECCT being electrically connected to console connector CC via second electrical connector SEC and ultrasound transducer connector UTC being electrically connected to first electrical connector FEC, based on a value of an impedance measured between the two measurement terminals TM₁, TM₂. Additional measurement terminals may be used in a similar manner.

Electrical cable ECAB illustrated in Fig. 2 may optionally include first electrical conductor FCON and second electrical conductor SCON which are suitable for providing a potential difference from the console connector CC to the electrical circuit ECCT by means of corresponding terminals in the console connector and the second electrical connector. Optionally, electrical cable ECAB may also include shield SH which is configured to shield each electrical conductor SCON, SCON. Shield SH may for example cover first electrical conductor FCON and second electrical conductor SCON in the form of a sheath. Optionally, shield SH may be electrically connected to one of the first electrical conductor FCON and the second electrical conductor SCON in order to provide such electrical shielding. Such electrical shielding and conductors may also be used in electrical cable ECAB illustrated in Fig. 1.

In one implementation, extension cable EXC2 illustrated in Fig. 2 may optionally include a memory MEM. Memory MEM is preferably a non-volatile memory. Non-volatile random access memory, i.e. NVRAM is one suitable example. Other suitable examples include flash memory Storage such as electrically erasable programmable read-only memory, i.e. EEPROM, solid-state drive, i.e. SSD, NAND, and so forth. Memory MEM may be used to store a count value indicative of a) a number of times that a corresponding ultrasound transducer connector UTC has been connected to the first electrical connector FEC based a number of times the voltage level of the multiple state output MSOP transitions to the voltage level V₃ corresponding to iii) the electrical circuit ECCT being electrically connected to the console connector CC via the second electrical connector SEC and the ultrasound transducer connector UTC being electrically connected to the first electrical connector FEC, and/ or b) a number of times that a corresponding console connector CC has been connected to the second electrical connector SEC based on a number of times the voltage level of the multiple state output MSOP transitions to the voltage level V₂ corresponding to ii) the electrical circuit ECCT being electrically connected to the console connector CC via the second electrical connector SEC and the ultrasound transducer connector UTC not being electrically connected to the first electrical connector FEC.

Memory MEM may thus store number of connection cycles between one or both of the extension cable's connectors and their corresponding ultrasound transducer and console connectors. Advantageously counts of these connection cycles may be used to indicate the need to replace extension cable EXC2. In so doing the reliability of a system that uses extension cable EXC2 may be improved.

In this implementation the count value(s) for a) and b) above may optionally be determined by a counter within electrical circuit ECCT. Alternatively these count value(s) may be determined in a separate console when the console is connected to second electrical connector SEC via console connector CC. The latter configuration has the benefit of reduced complexity of extension cable EXC2. In this latter configuration electrical cable ECAB may optionally also include third electrical conductor TCON that is in communication with electrical circuit ECCT for providing voltage levels V₁, V₂, V₃ of multiple-state output MSOP to the console by means of a corresponding terminal in each of the second electrical connector SEC and the console connector CC. The third electrical conductor TCON may also be in further communication with memory MEM for receiving the count value from the console CS. Thus, in this implementation, third electrical conductor TCON acts as a bidirectional signal path. Using a bidirectional signal path in this manner reduces the number of electrical conductors in electrical cable ECAB and thereby reduces its weight and improves its flexibility. Additional conductors such as optional conductor VCON illustrated in Fig. 2 may also be included within electrical cable ECAB. Such conductors may be used in alternative implementations to, for example, provide separate signal paths for communicating multiple state output MSOP to the console and for receiving the count value from the console CS, for communicating ultrasound signals between first electrical connector FEC and second electrical connector SEC, or for other purposes.

Optionally, electrical circuit ECCT may be provided with a housing HOU. Electrical circuit ECCT may be disposed within housing HOU and first electrical connector FEC may be attached to housing HOU to provide structural support to first electrical connector FEC.

Optionally, as illustrated in Fig. 2, electrical circuit ECCT may also include an amplifier AMP. Amplifier AMP may for example be a charge amplifier or a current amplifier or a voltage amplifier. In one specific implementation the amplifier is a differential charge amplifier. Amplifier AMP may be used to amplify electrical signals received from an ultrasound transducer by means of first electrical connector FEC and ultrasound transducer connector UTC. The amplified output may subsequently be communicated to second electrical connector SEC by means of one or more conductors such as electrical conductor VCON. In so doing, the integrity of the ultrasound signals may be preserved following their transmission along the extent of electrical cable ECAB. Thereto, as illustrated in Fig. 2, first electrical connector FEC may include two transducer terminals TT₁, TT₂ for receiving and/ or transmitting electrical signals via corresponding terminals TT'₁, TT'₂ in ultrasound transducer connector UTC. Amplifier AMP is in electrical communication with transducer terminals TT₁, TT₂ for amplifying the electrical signals. Additional transducer terminals may also be included in connectors UTC and FEC in a similar manner. Electrical cable may also include one or more electrical conductors, such as electrical conductor VCON, for transmitting amplified signals to second electrical connector SEC. Such electrical signals maybe further processed by a console, not illustrated in Fig. 2, when connected to second electrical connector SEC via console connector CC.

In one exemplary application extension cable EXC1, EXC2 may be used in an ultrasound-based position tracking system. In this application, ultrasound transducer connector UTC is electrically connected to an ultrasound transducer. The ultrasound transducer may be a detector connected to ultrasound transducer connector UTC via transducer terminals TT₁', TT₂' such that when ultrasound transducer connector UTC is connected to first electrical connector FEC, and when second electrical connector SEC is connected to console connector CC, amplifier AMP amplifies detected ultrasound signals and transmits these via amplifier AMP and electrical cable ECAB to console connector CC that is connected to a console. The console may be in communication with a beamforming ultrasound imaging probe that is configured to generate an ultrasound field, and include a processor configured to: provide a reconstructed ultrasound image corresponding to the ultrasound field of the beamforming ultrasound imaging probe and to compute a position of the ultrasound transducer of the ultrasound detector respective the ultrasound field based on ultrasound signals transmitted between the beamforming ultrasound imaging probe and the ultrasound transducer, and to provide an icon in the reconstructed ultrasound image based on the computed position of the ultrasound transducer. A suitable technique for processing the detected signals and determining the transducer position based on the time of flight of detected ultrasound signals and corresponding beam of the beamforming ultrasound imaging probe in which a maximum signal is detected is disclosed in more detail in document "A Non-disruptive Technology for Robust 3D Tool Tracking for Ultrasound-Guided Interventions" by Jay Mung, Francois Vignon, and Ameet Jain, in MICCAI 2011, Part I, LNCS 6891, pp. 153-160, 2011, A. Martel, and T. Peters (Eds.).

Fig. 6 illustrates a system SYS that includes extension cable EXC2, console CON and console connector CC. Console CON may optionally include the functionality of the aforementioned ultrasound-based position tracking system. System SYS comprising extension cable EXC2, console CON, and console connector CC. Console CON is connected to extension cable EXC2 via console connector CC and second electrical connector SEC. Console CON receives voltage levels V₁, V₂, V₃ via third electrical conductor TCON of extension cable EXC2, and shifts voltage level V₁ corresponding to i) the electrical circuit ECCT not being electrically connected to console connector CC via second electrical connector SEC by a predetermined voltage to an adjusted voltage level V'₁. A voltage level shifter or pullup resistor may for example be used to provide this voltage shift. Console CON also includes counter CTR that generates a count value indicative of a) a number of times that corresponding ultrasound transducer connector UTC has been connected to first electrical connector FEC, and/ or b) a number of times that corresponding console connector CC has been connected to second electrical connector SEC. Counter CTR may be a dedicated counter integrated circuit or its functionality may be carried out by a processor. The count value(s) of counter CTR are based on voltage levels V₁, V₂, V₃ and adjusted voltage level V'₁. Counter CTR also provides the count value(s) to memory MEM of electrical circuit ECCT via third electrical conductor TCON and console connector CC.

In so doing, a reliable system is provided since the need to replace the extension cable can be assessed by reading the value(s) in memory MEM.

With further reference to Fig. 6, console CON of system SYS may further include processor PROC. Processor PROC includes instructions which when executed by processor PROC cause processor PROC to process electrical signals received and/ or transmitted between processor console CON and amplifier AMP of electrical circuit ECCT. The instructions cause the processor PROC to process the electrical signals if the count value meets a first count condition, and to suspend processing of the electrical signals if the count value meets a second count condition.

By suspending processing after, for example the count value has exceeded a predetermined value, reliability of the system can be improved because the user is alerted to the need to replace extension cable ESC2.

In an alternative implementation an arrangement is provided. The arrangement includes extension cable EXC1 or EXC2 of Fig. 1 or Fig. 2 respectively, console CON, and console connector CC. Console CON further includes processor PROC comprising instructions which when executed on processor PROC cause processor PROC to determine a status of a connection between first electrical connector FEC and corresponding ultrasound transducer connector UTC based on the actual voltage level of the multiple-state output MSOP. Processor PROC is further configured to i) indicate said status, e.g. to a user and/ or ii) process electrical signals received and/ or transmitted between the processor and the amplifier AMP of the electrical circuit ECCT based on said connection status. The present, or current connection status may for example be indicated on a display, for example as an icon or alternatively via an indicator lamp such as a light emitting diode. In the aforementioned tracking application in which the position of an ultrasound transducer is indicated respective an ultrasound image, the display of the icon may for example be suspended if it is determined that the present status corresponds to connection state ii); i.e. electrical circuit ECCT being electrically connected to console connector CC via second electrical connector SEC and ultrasound transducer connector UTC not being electrically connected to first electrical connector FEC. Advantageously this reduces the risk of misinterpreting the ultrasound transducer position when ultrasound transducer connector UTC is not electrically connected to first electrical connector FEC.

In summary, an extension cable has been provided. The extension cable includes a first electrical connector for connecting to a corresponding ultrasound transducer connector, a second electrical connector for connecting to a corresponding console connector, an electrical circuit, and an electrical cable. The electrical circuit provides a multiple-state output, the multiple states being voltage levels corresponding to each of i) the electrical circuit not being electrically connected to the console connector, ii) the electrical circuit being electrically connected to the console connector and the ultrasound transducer connector not being electrically connected to the first electrical connector, and iii) the electrical circuit being electrically connected to the console connector and the ultrasound transducer connector being electrically connected to the first electrical connector.

Various embodiments and options have been described in relation to the extension cable, and it is noted that the various embodiments may be combined to achieve further advantageous effects. Any reference signs in the claims should not be construed as limiting the scope of the invention

Any of the method steps disclosed herein may be recorded in the form of instructions which when executed on a processor cause the processor to carry out such method steps. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor "DSP" hardware, read only memory "ROM" for storing software, random access memory "RAM", non-volatile storage, etc. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray™ and DVD.

## Claims

1. An extension cable (EXC1, EXC2) for an ultrasound system, the extension cable comprising:
a first electrical connector (FEC) for connecting to a corresponding ultrasound transducer connector (UTC);
a second electrical connector (SEC) for connecting to a corresponding console connector (CC);
an electrical circuit (ECCT); and
an electrical cable (ECAB);
wherein the electrical circuit (ECCT) is disposed between the first electrical connector (FEC) and the electrical cable (ECAB), and wherein the electrical cable (ECAB) is disposed between the electrical circuit (ECCT) and the second electrical connector (SEC) for extending an electrical connection between the ultrasound transducer connector (UTC) and the console connector (CC); and
wherein the electrical circuit (ECCT) is configured to provide a multiple-state output (MSOP), the multiple states being voltage levels (V₁, V₂, V₃) corresponding to each of i) the electrical circuit (ECCT) not being electrically connected to the console connector (CC) via the second electrical connector (SEC), ii) the electrical circuit (ECCT) being electrically connected to the console connector (CC) via the second electrical connector (SEC) and the ultrasound transducer connector (UTC) not being electrically connected to the first electrical connector (FEC), and iii) the electrical circuit (ECCT) being electrically connected to the console connector (CC) via the second electrical connector (SEC) and the ultrasound transducer connector (UTC) being electrically connected to the first electrical connector (FEC).

2. The extension cable (EXC2) according to claim 1 wherein the first electrical connector (FEC) further comprises at least two measurement terminals (TM₁, TM₂) for connecting to corresponding terminals (TM'₁, TM'₂) in the ultrasound transducer connector (UTC);
and wherein the electrical circuit (ECCT) is configured to provide the voltage level (V₃) corresponding to iii) the electrical circuit (ECCT) being electrically connected to the console connector (CC) via the second electrical connector (SEC) and the ultrasound transducer connector (UTC) being electrically connected to the first electrical connector (FEC), based on a value of an impedance measured between the at least two measurement terminals (TM₁, TM₂).

3. The extension cable (EXC2) according to claim 1 or claim 2 wherein the electrical cable (ECAB) further comprises a first electrical conductor (FCON) and a second electrical conductor (SCON) for providing a potential difference from the console connector (CC) to the electrical circuit (ECCT) by means of corresponding terminals in the console connector and the second electrical connector.

4. The extension cable (EXC2) according to claim 3 wherein the voltage level (V₁) corresponding to i) the electrical circuit (ECCT) not being electrically connected to the console connector (CC) via the second electrical connector (SEC), is provided if the first electrical conductor (FCON) an the second electrical conductor (SCON) is not electrically connected to its corresponding terminal in the console connector (CC).

5. The extension cable (EXC2) according to claim 3 wherein the electrical cable (ECAB) further comprises a shield (SH) configured to shield each electrical conductor (SCON, SCON) of the electrical cable, and wherein the shield (SH) is electrically connected to one of the first electrical conductor (FCON) and the second electrical conductor (SCON).

6. The extension cable according to claim 1 wherein the electrical circuit (ECCT) comprises at least one active electronic component (TR₁) configured to operate as an electrical switch;
wherein the electrical switch comprises:
an input (IP) configured to detect whether the ultrasound transducer connector (UTC) is electrically connected to the first electrical connector (FEC); and
an output (OP) configured to provide the multiple-state output (MSOP).

7. The extension cable according to claim 1 wherein the electrical circuit comprises only passive electrical components.

8. The extension cable (EXC2) according to claim 1 wherein the electrical circuit (ECCT) further comprises a memory (MEM) configured to store a count value indicative of a) a number of times that a corresponding ultrasound transducer connector (UTC) has been connected to the first electrical connector (FEC) based a number of times the voltage level of the multiple state output (MSOP) transitions to the voltage level (V₃) corresponding to iii) the electrical circuit (ECCT) being electrically connected to the console connector (CC) via the second electrical connector (SEC) and the ultrasound transducer connector (UTC) being electrically connected to the first electrical connector (FEC), and/ or b) a number of times that a corresponding console connector (CC) has been connected to the second electrical connector (SEC) based on a number of times the voltage level of the multiple state output (MSOP) transitions to the voltage level (V₂) corresponding to ii) the electrical circuit (ECCT) being electrically connected to the console connector (CC) via the second electrical connector (SEC) and the ultrasound transducer connector (UTC) not being electrically connected to the first electrical connector (FEC).

9. The extension cable according to claim 8 wherein the electrical cable (ECAB) further comprises a third electrical conductor (TCON) in communication with the electrical circuit (ECCT) for providing the voltage levels (V₁, V₂, V₃) of the multiple-state output (MSOP) to the console by means of a corresponding terminal in each of the second electrical connector (SEC) and the console connector (CC); and wherein the third electrical conductor (TCON) in further communication with the memory (MEM) for receiving the count value from the console (CS).

10. The extension cable (EXC2) according to any one of claims 1 - 9 further comprising a housing (HOU);
wherein the electrical circuit (ECCT) is disposed within the housing (HOU) and the first electrical connector (FEC) is attached to the housing (HOU).

11. The extension cable (EXC1, EXC2) according to any one of claims 1 - 9 wherein the ultrasound system is an ultrasound-based position tracking system and wherein the ultrasound transducer connector (UTC) is electrically connected to an ultrasound transducer.

12. The extension cable according to any one of claims 1 - 9 wherein the first electrical connector (FEC) includes at least two transducer terminals (TT₁, TT₂) for receiving and/ or transmitting electrical signals via corresponding terminals (TT'₁, TT'₂) in the ultrasound transducer connector (UTC), and wherein the electrical circuit (ECCT) further comprises a amplifier (AMP) in electrical connection with the at least two transducer terminals (TT₁, TT₂) for amplifying the electrical signals.

13. System (SYS) comprising the extension cable (EXC2) according to any one of claims 9 - 12;
a console (CON); and
a console connector (CC);
wherein the console (CON) is connected to the extension cable (EXC2) via the console connector (CC) and the second electrical connector (SEC);
wherein the console is configured to receive the voltage levels (V₁, V₂, V₃) via the third electrical conductor (TCON) of the extension cable (EXC2), and to shift the voltage level V₁ corresponding to i) the electrical circuit (ECCT) not being electrically connected to the console connector (CC) via the second electrical connector (SEC) by a predetermined voltage to an adjusted voltage level V'₁,
wherein the console (CON) comprises a counter (CTR) configured to generate the count value based on the voltage levels (V₁, V₂, V₃) and the adjusted voltage level V'₁, and wherein the counter (CTR) is further configured to provide the count value to the memory (MEM) of the electrical circuit (ECCT) via the third electrical conductor (TCON) and the console connector (CC).

14. System (SYS) according to claim 13 wherein the console (CON) further comprises a processor (PROC) comprising instructions which when executed by the processor (PROC) cause the processor (PROC) to process electrical signals received and/ or transmitted between the console (CON) and the amplifier (AMP) of the electrical circuit (ECCT); and
wherein the instructions cause the processor (PROC) to process the electrical signals if the count value of the counter meets a first count condition, and to suspend processing of the electrical signals if the count value meets a second count condition.

15. Arrangement comprising the extension cable according to any one of claims 1 - 12;
a console (CON); and
a console connector (CC);
wherein the console (CON) further comprises a processor (PROC) comprising instructions which when executed on the processor (PROC) cause the processor (PROC) to determine a status of a connection between the first electrical connector (FEC) and the corresponding ultrasound transducer connector (UTC) based on the actual voltage level of the multiple-state output (MSOP), and wherein the processor (PROC) is further configured to i) indicate said status and/ or ii) process electrical signals received and/ or transmitted between the processor and the amplifier (AMP) of the electrical circuit (ECCT) based on said connection status.
